Europäisches Patentamt

European Patent Office

Office européen des brevets

(19)

(11) Publication number: **0 314 439**
A2

(12)

# EUROPEAN PATENT APPLICATION

(21) Application number: **88310033.1**

(22) Date of filing: **25.10.88**

(51) Int. Cl.⁴: **C 12 N 1/04**
//(C12N1/04,C12R1:645)

(30) Priority: **26.10.87 US 113007   03.12.87 US 128394**

(43) Date of publication of application:
**03.05.89 Bulletin 89/18**

(84) Designated Contracting States:
**AT BE CH DE ES FR GB GR IT LI LU NL SE**

(71) Applicant: **NATIVE PLANTS INCORPORATED**
**417 Wakara Way**
**Salt Lake City Utah 84108  (US)**

(72) Inventor: **Wood, Timothy Eldridge**
**1200 Lake Street**
**Salt Lake City Uta 84104  (US)**

**Brown, Michael Steven**
**1260 Alameda Avenue No. 1**
**Salt Lake City Uta 84102  (US)**

(74) Representative: **Goldin, Douglas Michael et al**
**J.A. KEMP & CO. 14, South Square Gray's Inn**
**London WC1R 5EU  (GB)**

(54) Microbial inoculants and methods for producing same.

(57) The present invention relates to a method for producing dry, shelf-stable microbial inoculants comprising viable microorganisms which have a degree of dryness selected to prevent undue contamination of said inoculant and premature germination of said microoganisms which includes combining the microorganisms and a water absorbent/carrier material at a rate sufficient to attain the selected degree of dryness while maintaining desired viability.

**Description**

## MICROBIAL INOCULANTS AND METHODS FOR PRODUCING SAME

## FIELD OF THE INVENTION

The present invention relates to the preparation and use of microorganisms for soil or plant inoculants. Specifically, the present invention relates to a method for producing a dry, shelf-stable microbial inoculant which maintains viability over long periods at room temperature and is thus useable to improve the growth and survival of economically important plants.

## BACKGROUND OF THE INVENTION

1. Description of Related Art

Publications and other materials used to illuminate the Background, Summary, and Detailed Description of the Invention disclosed in this Specification are incorporated herein by reference and are numerically referenced in the following text and respectively grouped in the appended Bibliography.

Mycorrhizas are symbiotic associations between beneficial soil fungi and the roots of higher plants (1,2). The fungi colonize plant roots, extend their hyphae (thread-like filaments) into surrounding soils and form a fundamentally important linkage between the plant and its belowground environment. The fungus obtains from its host nutrient compounds that are required for growth. The plant in turn benefits from the association through improved uptake of phosphorus, trace metals and other sparingly soluble nutrients (3,4), enhanced resistance to many root pathogens (5,6) and generally 20-200% greater growth, yield and survival (7,8). Approximately 98% of all higher plants form mycorrhizas and most require the association for optimal performance.

The fungi involved in mycorrhizal associations are termed mycorrhizal fungi. Several groups of these organisms have been delineated. The most widespread group comprises vesicular-arbuscular mycorrhizal (hereinafter "VAM") fungi. These organisms have extremely broad host ranges. They associate with 90-95% of all higher plant species including most agricultural and horticultural crops and many valuable tree species (7, 8).

Because of their ability to improve the growth, yield, and survival of many commercially important plant species, VAM fungi hold considerable potential for use in agriculture, forestry, reclamation, and horticulture. In such uses, the fungi are typically mixed with carrier material to facilitate delivery, and are then applied as inoculants to soils or seeds.

A variety of inoculant formulations and carrier materials have been used in past attempts to formulate practicable microbial inoculants. Soil, clays, sand, coal, kaolin powder, sphagnum, peat, polyacrylimides, hydrophylic polymers, oil based carriers, and cellulose have all been used to deliver microorganisms into soil (9-21). Some inoculants have been prepared in moist form in which case they typically must be stored under refrigeration to prevent spoilage and premature spore germination. Others have been dried, in which case they may be conveniently stored at room temperature.

Despite such attempts, VAM fungal inoculants have not been widely commercialized, in part because practicable carrier systems have not been developed. Carrier materials have been too expensive, harmful to target plants or microorganisms, not widely or consistently available (18), and/or too bulky for convenient shipping and use. Other formulations have proven inconsistent in performance, because of variation in microbial viability following drying, shipping, and/or storage.

To be commercially desireable and of greatest utility, therefore, microbial inoculants should be formulated with carriers that are: (a) relatively light weight to promote ease in distribution and use; (b) non-toxic to microorganisms and target plants; (c) widely available and relatively inexpensive; and (d) suitable in structure to facilitate application through farm machinery. Moreover, carriers should be compatible with modes of manufacture that promote the maintenance of high levels of microbial viability during inoculant formulation and storage.

As a result of the invention described and claimed herein, microbial inoculants with superior shelf-life, shipping, and application characteristics have been developed. The inoculants are relatively light in weight, they are dry and they do not require refrigeration to prevent spoilage or premature spore germination during storage. Uniform granular consistencies allow metered application in nurseries and agricultural fields using standard agricultural equipment. Moreover, the carrier materials may be inexpensive and thus this system is amenable to production of plant and soil inoculants on a commercial scale. And, importantly, the carrier and formulation systems of the present invention provide for controlled inoculant drying at defined rates that maintain high levels or microbial viability during production and storage. Use of this process obviates the need for expensive humidity control and forced air equipment to control drying.

EP 0 314 439 A2

## SUMMARY OF THE INVENTION

The present invention provides a novel means for formulating and producing dry, shelf-stable soil and plant inoculants comprising microorganisms and carrier materials. The invention is applicable to a wide variety of VAM fungal species and to other microorganisms. Embodiments of the invention use absorbent materials, such as calcined and non-calcined clay granules, as both carriers and/or drying agents for microorganisms. Use of such materials allows for controlled drying of inoculant formulations at rates necessary to maintain high levels of microbial viability.

Specifically, this invention relates to materials and methods for producing dry, shelf-stable, inoculants of microorganisms that are useful for improving plant growth and survival. The inoculants comprise viable microorganisms or propagules and carrier material. The drying rate which ensures maintenance of maximum or other desired viability is determined for each particular microorganism as described herein. Additions of drying agent (which may or may not also be used as a carrier) based on calculated dessication schedules are then used to carefully and uniformly dry the inoculant. Thus, this novel process can be used to control the rate of inoculant drying to ensure maximum microbial viability.

## BRIEF DESCRIPTION OF THE DRAWINGS

Figure 1 is a graph showing matric potential versus moisture content curves for the absorbent granular materials attapulgite and Turface®. The curves show the water holding capacity of the materials tested at matric potentials of between -0.1 MPa and -1000.0 MPa.

Figure 2 shows the semi-log drying plots of VAM propagules from -0.26 MPa to -50 MPa that extend over periods of 3, 7, and 14 days.

## DETAILED DESCRIPTION OF THE INVENTION

This invention involves the production of dry, shelf-stable, microbial inoculants used for improving plant growth and survival. The production process includes several stages. The preferred drying rate, i.e., the drying time to a predetermined level of dessication that maintains a desired level of microbial viability, is determined for the microorganism to be used. Independently, absorbent material to be used as the drying agent and/or carrier for the inoculant is characterized with respect to matric potential as a function of moisture content. The microorganisms are then mixed with a small amount of this absorbant material and additional absorbant material is added on a schedule calculated to dry the microorganisms at the selected drying rate. Thus, the drying rate is maintained by addition of desiccant, which may also serve as a carrier, until the desired state of desiccation is attained.

The following description provides details of the manner in which an embodiment of the present invention may be made and used in order to produce a dry, shelf-stable, microbial inoculant for use in improving plant growth and survival. This description, while exemplary of the present invention, is not to be construed as specifically limiting the invention. Such variations which would be within the purview of one skilled in this art are to be considered within the scope of this invention, which is limited only by the hereafter appended claims.

## EXPERIMENTAL

## Production of Vesicular-Arbuscular Mycorrhizal Inoculants

As described in detail below, the drying rate of VAM fungal propagules produced was selected to ensure their maximum viability. Fungal propagules were then mixed with absorbant carrier materials to provide for drying at that drying rate. Resultant VAM inoculants remained viable and effective for periods in excess of six months.

1. Production of fungal propagules.
VAM fungal propagules were produced in association with roots of living plants in pots in a greenhouse (22) or in association with tissue cultured roots in sterile containers (23, 24). Propagules suitable for use in this invention include, but are not limited to, chlamydospores ("spores") and VAM root fragments.

Spores and roots were removed from the culture substrates. VAM root systems were chopped into suitable fragments in a blender. If the propagules of the VAM fungal species of interest were produced in clusters or sporocarps, as are those of G. intraradices and G. deserticola, the clusters were broken up by blending in a Waring blender at high speed for 10-60 seconds, followed by sieving to separate the individual spores and

3

small clusters from coarser and finer debris. VAM fungal species that produce spores singly, such as those of G. etunicatum and Gigaspora margarita, did not require a blending step. The spore and root preparations were surface disinfested to remove microbial contaminants using the methods of Tommerup and Kidby (25).

2. Preparation and characterization of absorbent granular carriers/drying agents.

The fungal propagules were mixed with an absorbent drying agent and dried at the rates selected as described infra. The absorbent drying agent was also used herein as a carrier for the fungal propagules in order to facilitate application of the inoculant to plants and soils. If the drying agent is unsuitable as carrier material, the dried propagules may be separated from the drying agent and applied to a suitable carrier.

Many relatively inert absorbent materials are suitable for use as drying agents/carriers in this processing system. Suitable materials include, but are not limited to, vermiculite, attapulgite, Turface® (a calcined montmorillinite clay), and zeolite. Some agricultural waste materials, such as corncob grit, can also be used, but these are subject to rapid spoilage following rewetting in soils.

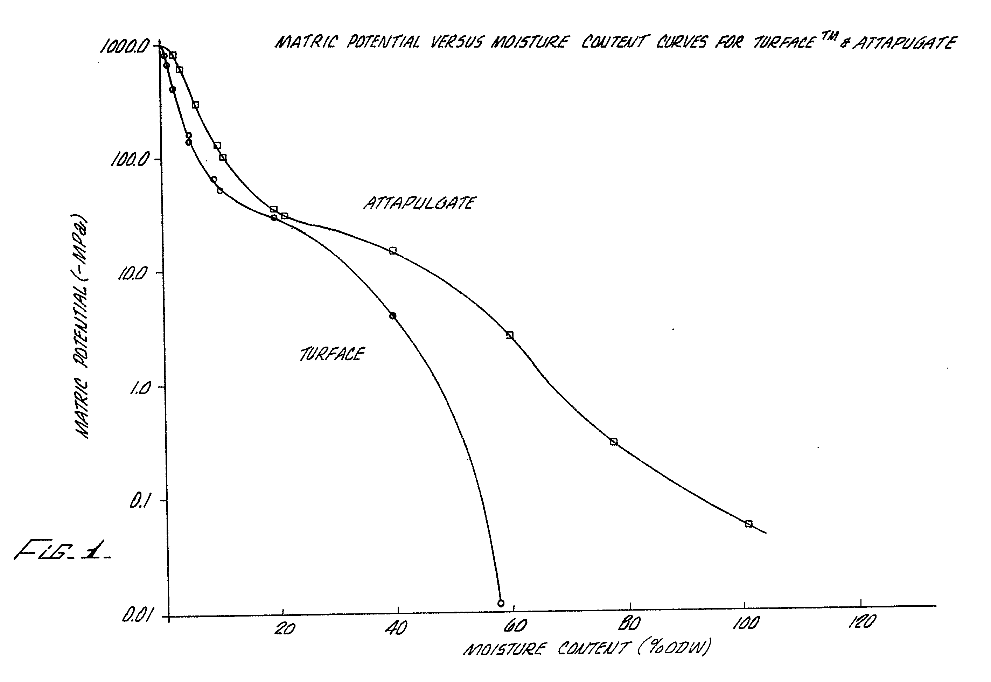

Drying agent/carrier materials were characterized with respect to matric potential as a function of moisture content. This measurement was used to determine the degree to which the granular material absorbed moisture and, thus, would dry the propagules. This characterization was accomplished using the filter paper method of Fawcett and Collis-George (26) and Hamblin (27). Matric potential versus moisture content curves were established by this method for the matric potential range -0.1 to -1,000 MPa. Examples of such curves are given for attapulgite and Turface® in Figure 1.

3. Measurement of spore germination as a function of drying rate and determination of preferred drying rate.

The degree to which microorganisms were dried by the process described herein was chosen with regard to maintenance of viability, prevention of premature germination, and prevention of spoilage. In the present example, VAM fungi were dried to matric potentials of between -50 and -100 MPa, which is in excess of the minimum dryness necessary to fulfill the above functional criteria, but nevertheless act as a safeguard without detrimental effect (See 28, 29). This drying was done without significant loss of spore viability, and at these matric potentials spores did not germanate prematurely. With respect to contamination and spoilage, it is known that competitive growth for most potentially contaminating bacteria and actinomycetes ceases at about -4.0 MPa and that growth of many airborne and potentially contaminating fungi (e.g., Fusarium spp., Penicillium spp., and Aspergillus spp.) cease in the range from about -10 MPa to about -40 MPa.

The matric potential range of about -50 MPa to about -100 MPa used in the present example represents an operationally effective level of dryness for use with this invention. It is exemplary, however, and is not to be construed as specifically limiting the invention. Spores of VAM fungi can be dried to matrix potentials well below -100 MPa (e.g., - 300 MPa and drier) without significant loss of viability, and in practice, inoculants could be dried to these levels. It may also be possible to dry inoculants to lesser extents (e.g., in the range from about -20 to about -50 MPa) without significant risk of spoilage, particularly when very inert carriers, which will not support significant microbial growth, are used.

While VAM fungal spores can be dried to matrix potentials in excess of -50 MPa without significant loss of viability, spore survival is dependent on rates of desiccation. If drying is conducted too rapidly, propagules may be killed. If drying is conducted too slowly, propagules may lose effectiveness through contamination or premature germination. The preferred drying rate, and the one used herein, was the minimum drying time to between about -50 MPa and about -100 MPa with which maximum propagule viability was maintained, viability being measured in terms of propagule germination.

Preferred drying rates were determined for individual VAM fungal species as follows. Propagules of a given VAM fungus were mixed into a sand loam matrix with a known matric-potential-versus-moisture-content function, as determined by the filter paper method noted above (26, 27). The sand-loam material was adjusted beforehand to a matric potential of -0.1 MPa. Approximately 500 grams of the moist spore and sand-loam mixture were then dispensed into a 500 ml specimen jar. The mouth of the jar was covered with a sheet of mira-cloth and then with the jar lid which was perforated to allow evaporative drying. The drying rate was controlled by the total cross sectional area of the holes in the lid. Drying to -100 MPa was accomplished in 3-4 days when holes in the jar lid totaled 45.4 cm$^2$, in 6-8 days when holes totaled 6.8 cm$^2$, and in 14-16 days when holes totaled 2.3 cm$^2$ in area.

For each VAM fungal species, several jars with differing evaporative drying rates were set up. In this way, the effects of drying rate on spores survival could eventually be studied.

Jars containing the spore mixture were then sealed in one liter cylindrical canisters along with an olephan bag containing 750 grams of oven dried (at 200°C) drierite. The drierite provided a sink for moisture defusing out of the jar of inoculum. Canisters thus assembled were placed on a roller table set at low speed such that the canisters rotated at about two revolutions per minute. The table was further fitted with a timer such that it was activated for two minutes each half hour.

Rates of drying were monitored by measuring weight loss from the jars. Drying was continued until moisture levels corresponding to a matric potential of about -100 MPa were attained.

More rapid dessication (e.g., from -0.1 MPa to -100 MPa in 2-3 hours) was accomplished by spreading the spore mixture into a thin layer and air drying on a bench top.

When dry, spores were separated from the matrix material and tested for survival, measured as percent germination after 14 days (28). Levels of germination were then examined as functions of drying rate in order to define preferred drying rates.

Table 1 presents results from one such series of drying experiments with isolates of several species of VAM fungi. These data show the percent of germination of VAM fungal spores assayed following drying in sand-loam substrates to a matric potential of -100 MPa and storage at room temperature for 1 mo. and for 5-6 mo. Results for the Glomus intraradices isolate show that spore germination declined markedly if drying was accomplished over a 2-3 hour period (Table 1). Declines were less pronounced if spores were dried over 3-4 days. With drying over 6-8 or 14-16 days, spore germination remained comparable to fresh materials. Glomus deserticola isolate spores were more sensitive to desiccation and required slower drying over 14-16 days to prevent significant loss of germination. Glomus etunicatum isolate spores survived rapid drying (2-3 hours) and even showed enhanced germination following such treatment. The above results indicate that preferred drying rates are: for the G. intraradices isolate, 6-8 days; for the G. deserticola isolate, 14-16 days; and, for the G. etunicatum isolate, 1-3 hours. Optimum drying rates, as demonstrated herein, may vary markedly between species. Of course, the results in Table 1 and the preferred drying rates noted above may also be isolate-specific. Different isolates of G. intraradices, for example, may vary in response to drying and have differeing preferred drying rates.

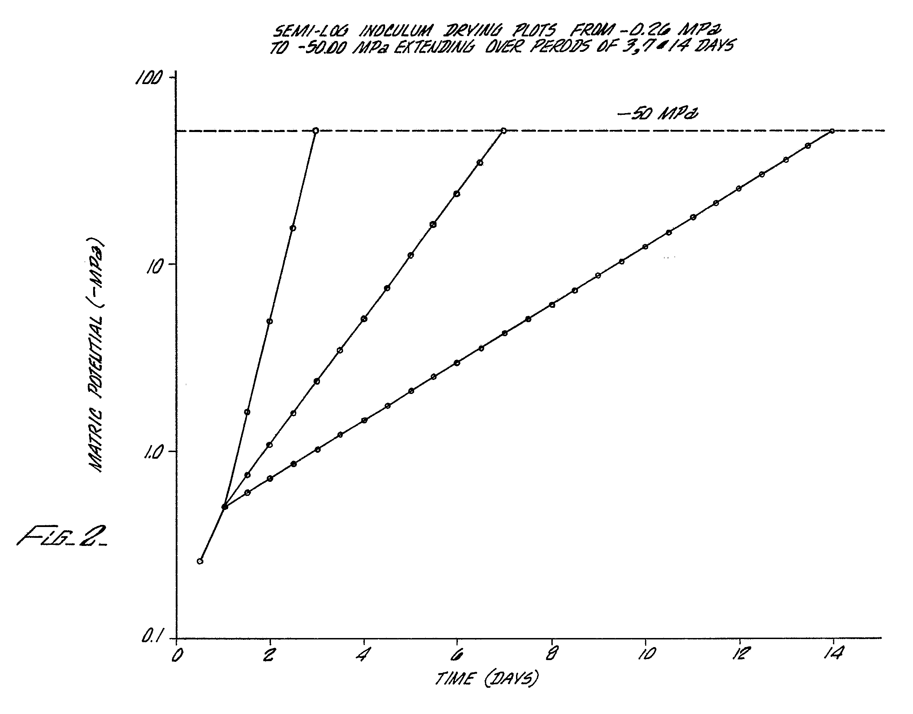

Preferred drying rates were next translated into operational inoculum drying schedules by plotting desired changes in inoculum matrix potential as functions of time. Figure 2 shows examples of three such drying schedules corresponding to preferred drying rates of 3 days, 7 days, and 14 days. These plots were used, as described below, to calculate inoculum formulation schedules.

The linear, semi-log plots shown in Figure 2 closely resemble the actual drying schedules measured for spores dried in the dessication jars described above. In practice, a variety of non-linear drying functions, with the same overall drying times of 3, 7 and 14 days, could be plotted and tested.

## 4. Addition of Propagules to Drying Agent/Carrier.

Spores of a VAM fungal species, for example, G. intraradices, G. deserticola, or G. etunicatum, were suspended in water and then mixed into a small volume of absorbent clay carrier. If desired, the propagules may be suspended in a dilute (e.g., 2%) solution of carboxymethyl cellulose to promote adhesion of the spores to the surface of the granular material. Volumes of suspension and carrier material were adjusted such that the solution nearly saturated the carrier material and allowed the fungal propagules to be evenly distributed over the surfaces of the carrier particles.

## 5. Drying of Inoculant by Addition of Absorbant Carrier.

Drying of this spore/carrier preparation was accomplished by addition of further absorbent carrier material, as described below, to draw moisture from the particles coated with propagules. Thus, the percent moisture content of the inoculant was reduced by increasing the bulk of the absorbent carrier material in the formulation. By this method, inoculant drying rates were regulated by controlling rates of addition of fresh, dry carrier material.

Schedules for inoculant formulation were calculated as follows using the optimum drying rates for the target fungal species and the matric potential-moisture content relationships of the desired carrier material. As noted, Figure 2 shows semi-log inoculum drying plots from -0.26 MPa to -50 MPa that extend over periods of 3, 7, and 14 days. From this Figure, desired changes in matric potential were read at half-day (12 hour) intervals. Corresponding 12 hour changes in moisture content for the desired carrier were then read from the matric potential- moisture content curves shown in Figure 1 for the appropriate matrix material. Finally, schedules for addition of dry carrier material were calculated from the desired changes in percent moisture using the relationship:

$$\text{Carrier Addition(g)} = 100 \times \text{Moisture Weight(g)} \times \frac{[\text{Current \% moisture} - \text{Desired \% moisture}]}{\text{Current \% moisture} \times \text{Desired \% moisture}]}$$

Tables 2a, 2b and 2c show inoculum formulation schedules for the 3, 7 and 14 day drying regimens plotted in Figure 2. The drying schedules are specific for the attapulgite carrier characterized in Figure 1. In this example, we chose to begin formulation by suspending VAM fungal spores in 320 grams of water and added this suspension to 400 grams of attapulgite for an initial moisture content of 80%. Subsequent additions of attapulgite were made twice each day according to the schedules shown. These 3, 7 and 14 day schedules are suitable for G. etunicatum, G. intraradices and G. deserticola, respectively.

## 6. Shelf-life of prepared VAM inoculants.

Shelf-life trials and bioassays of inoculants formulated using the methods given above show that inoculants remain viable and effective for periods in excess of six months.

The data presented in Table 3 are for Glomus intraradices inoculants prepared on attapulgite and Turface®

carriers, dried to -100 MPa, and stored at 4°C and 23°C for up to six months. Infectivity was tested in a bioassay for root colonization and was expressed as the percent of the host plant's root length that became colonized by the VAM fungus at two months following inoculation. Here, infectivity of the dried attapulgite inoculant compared favorably with the infectivity of control spores stored in moist sand at 4°C through 6 months. Turface® based inoculants proved more variable in performance and less effective in general. Thus, while there may be some variation with respect to the type of carrier used, these results show that the presently disclosed process of preparing mycorrhizal inoculants enables the dry storage of such inoculants at room temperature or under refrigeration without compromising viability and infectivity.

The data presented at Table 4 show plant growth responses to inoculation with Glomus intraradices inoculants prepared using the methods described herein on sand-loam, attapulgite, or Turface® carriers. The test plant was Trifolium subterraneum. Harvest was at seven weeks following sowing. Plants showed moderate growth increases when inoculated with sand-loam and attapulgite inoculants. Such increases most likely reflect responses to VAM development as the sand-loam and attapulgite carriers generally do not affect plant growth by themselves. The larger increases in plant growth found with Turface® based inoculants reflect both the effects of VAM inoculation and the presence of plant nutrients in Turface®.

### 7. Characteristics of VAM inoculants on granular carrier materials prepared by the described method.

Final inoculant preparations were comprised of about 20 percent granules coated with VAM fungal propagules and about 80 percent noncoated granules. The balance does not represent a significant waste in carrier material as the full inoculant bulk is typically useful in promoting uniform application of the product. The inoculants prepared by the presently disclosed method were dry and for the most part did not require refrigeration during storage to maintain high levels of infectivity.

The granular inoculants produced through the above methods are also relatively light in weight (bulk density = 0.6 g/cm for attapulgite and Turface® carriers), allowing for ease in handling and shipping. Furthermore, their uniform granular nature should allow metered application in nurseries and agricultural fields using standard equipment. The granular formulations have applications both as a direct soil amendment and in production of inoculant briquets for use with bareroot transplants.

Similar methods and means can be used to formulate powdered VAM inoculants with, for example, talc, clay, or diatomaceous earth carriers. Such powdered formulations may be used in seed coating and root-dip applications. We also note that the noncoated portion of the carrier material can be pretreated with fertilizer materials and growth promoting compounds to enhance root growth and encourage VAM colonization.

Although the foregoing invention has been described in detail by way of illustration and example for purposes of clarity of understanding, it will be understood that certain changes and modifications may be practiced within the scope of the appended claims.

Table 1

Germination of VAM Fungal Spores Following
Drying At Different Drying Rates

| VAM Fungus | Drying Time | Percent Spore Germination After Storage | |
|---|---|---|---|
| | | 1 mo | 5-6 mo |
| Glomus intraradices | Fresh | 79 ± 4 | -- |
| | 1-3 hr | 41 ± 2 | 25 ± 4 |
| | 3-4 d | 62 ± 3 | 62 ± 2 |
| | 6-8 d | 70 ± 8 | 77 ± 5 |
| | 14-16 d | 75 ± 3 | 70 ± 10 |
| Glomus deserticola | Fresh | 31 ± 3 | -- |
| | 1-3 hr | 1 ± 0 | 0 ± 0 |
| | 3-4 d | 1 ± 1 | 2 ± 2 |
| | 6-8 d | 1 ± 2 | 0 ± 1 |
| | 14-16 d | 37 ± 8 | 20 ± 5 |
| Glomus etunicatum | Fresh | 44 ± 1 | -- |
| | 1-3 hr | 60 ± 16 | 78 ± 9 |
| | 3-4 d | 68 ± 7 | 76 ± 9 |
| | 6-8 d | 73 ± 9 | 88 ± 6 |
| | 14-16 d | 53 ± 13 | 78 ± 3 |
| Glomus mosseae | Fresh | 21 ± 6 | -- |
| | 1-3 hr | 14 ± 3 | 15 ± 9 |
| | 3-4 d | 11 ± 5 | 14 ± 4 |
| | 6-8 d | 17 ± 0 | 23 ± 9 |
| | 14-16 d | 12 ± 1 | 24 ± 5 |
| Gigaspora margarita | Fresh | 36 ± 3 | -- |
| | 1-3 hr | 0 ± 0 | 0 ± 0 |
| | 23 d | 65 ± 13 | 75 ± 7 |

Table 2a

Three Day Semi-log Drying Schedule
for Attapulgite-based Inoculants
Appropriate for Use With Glomus etunicatum

| Day | Attapulgite Addition (g) | Moisture Addition (g) | % Moisture | Matric Potential (-MPa) |
|---|---|---|---|---|
| 1 | 400 | 320 | 80.0 | 0.3 |
| | 38 | -- | 73.0 | 0.5 |
| 2 | 62 | -- | 64.0 | 1.6 |
| | 87 | -- | 54.5 | 5.0 |
| 3 | 244 | -- | 38.5 | 16.0 |
| | 1169 | -- | 16.0 | 50.0 |

## Table 2b

Seven Day Semi-log Drying Schedule
for Attapulgite-based Inoculants
Appropriate for Use with
Glomus intraradices

| Day | Attapulgite Addition (g) | Moisture Addition (g) | % Moisture | Matric Potential (-MPa) |
|-----|--------------------------|------------------------|------------|--------------------------|
| 1 | 400 | 320 | 80 | 0.3 |
|   | 38  | -- | 73 | 0.5 |
| 2 | 19  | -- | 70 | 0.7 |
|   | 28  | -- | 66 | 1.1 |
| 3 | 15  | -- | 64 | 1.6 |
|   | 33  | -- | 60 | 2.3 |
| 4 | 19  | -- | 58 | 3.5 |
|   | 41  | -- | 54 | 5.0 |
| 5 | 47  | -- | 50 | 7.0 |
|   | 71  | -- | 45 | 11.0 |
| 6 | 110 | -- | 39 | 16.0 |
|   | 246 | -- | 30 | 23.0 |
| 7 | 533 | -- | 20 | 35.0 |
|   | 400 | -- | 16 | 50.0 |

Table 2c

Fourteen Day Semi-log Drying Schedule
for Attapulgite-based Inoculants
Appropriate for Use With
Glomus deserticola

| Day | Attapulgite Addition (g) | Moisture Addition (g) | % Moisture | Matric Potential (-MPa) |
|-----|-----|-----|-----|-----|
| 1 | 400 | 320 | 80.0 | 0.3 |
|  | 38 | -- | 73.0 | 0.5 |
| 2 | 13 | -- | 71.0 | 0.6 |
|  | 6 | -- | 70.0 | 0.7 |
| 3 | 10 | -- | 68.5 | 0.9 |
|  | 11 | -- | 67.0 | 1.0 |
| 4 | 7 | -- | 66.0 | 1.2 |
|  | 7 | -- | 65.0 | 1.4 |
| 5 | 12 | -- | 63.5 | 1.7 |
|  | 8 | -- | 62.5 | 2.0 |
| 6 | 13 | -- | 61.0 | 2.4 |
|  | 13 | -- | 59.5 | 2.9 |
| 7 | 14 | -- | 58.0 | 3.5 |
|  | 19 | -- | 56.0 | 4.2 |
| 8 | 16 | -- | 54.5 | 5.0 |
|  | 23 | -- | 52.5 | 6.0 |
| 9 | 27 | -- | 50.5 | 7.2 |
|  | 23 | -- | 48.5 | 8.5 |
| 10 | 36 | -- | 46.0 | 10.0 |
|  | 40 | -- | 43.5 | 12.0 |
| 11 | 54 | -- | 40.5 | 14.5 |
|  | 87 | -- | 36.5 | 17.5 |
| 12 | 108 | -- | 32.5 | 20.5 |
|  | 200 | -- | 27.0 | 25.0 |
| 13 | 270 | -- | 22.0 | 30.0 |
|  | 145 | -- | 20.0 | 35.0 |
| 14 | 178 | -- | 18.0 | 42.0 |
|  | 222 | -- | 16.0 | 50.0 |

Table 3

SHELF-LIFE TESTS FOR DRY GLOMUS INTRARADICES INOCULANTS

| Carrier | Storage Temp | Percent VAM Colonization After Given Inoculant Storage Period | | | |
|---------|--------------|--------|--------|--------|--------|
| | | 0 mo | 2 mo | 4 mo | 6 mo |
| Attapulgite | 23°C | 79 ± 10 | 90 ± 3 | 95 ± 4 | 87 ± 9 |
| | 4°C | 83 ± 8 | 96 ± 2 | 97 ± 2 | 94 ± 3 |
| Turface® | 23°C | 16 ± 19 | 76 ± 20 | 23 ± 26 | 0 ± 1 |
| | 4°C | 12 ± 17 | 81 ± 17 | 61 ± 31 | 30 ± 16 |
| Moist Sand | 4°C | 71 ± 11 | 95 ± 3 | 95 ± 4 | 97 ± 2 |

Table 4

Plant Growth Response to G. intraradices Inoculants

| Carrier | Storage Temperature (°C) | Plant Dry Weight (g) |
|---------|--------------------------|----------------------|
| Sand-Loam | 4 | 0.47 ± 0.09 |
| | 23 | 0.47 ± 0.06 |
| Attapulgite | 4 | 0.47 ± 0.07 |
| | 23 | 0.44 ± 0.05 |
| Turface® | 4 | 0.61 ± 0.08 |
| | 23 | 0.61 ± 0.16 |
| Control | -- | 0.34 ± 0.11 |

LITERATURE CITED

1) Harley, J.L. and S.E. Smith. 1983 Mycorrhial Symbiosis. Academic Press, New York. 483 pp.

2) Powell, C.L. and D.J. Bagyaraj (eds.). 1984. VA Mycorrhiza. CRC Press, Boca Raton. 234 pp.

3) Sanders, F.E., B. Mosse, and P.B. Tinker. 1975. Endomycorrhizas. Academic Press, New York. 626 pp.

4) Bowen, G.D. 1973. Mineral nutrition of ectomycorrhizae. pp. 151-205. In G.C. Marks and T.T. Kozlowski (eds.). Ectomycorrhizae. Academic Press, New York.

5) Marx, D.H. 1973. Mycorrhizae and feeder root disease. pp. 351-382. In G.C. Marks and T.T. Kozlowski (eds.). Ectomycorrhizae. Academic Press, New York.

6) Schonbeck, F. 1968. Effect of endotrophic mycorrhiza on disease resistance on higher plants. Z. Pflanzankr. Pflanzenschutz. 85: 191-196.

7) Menge, J.A. 1981. Mycorrhiza agriculture technologies. pp. 383-424. In Background Papers for Innovative Biological Technologies for Lesser Developed Countries. U.S. Government Printing Office, Washington, D.C.

8) Mosse, B. 1981. Vesicular-arbuscular Mycorrhiza Research for Tropical Agriculture. Hawaii Institute for Tropical Agriculture and Human Resources. Research Bulletin 194, 82 pp.

9) Dehne, H.W. and G.F. Backhaus. 1986. The use of vesicular-arbuscular mycorrhizal fungi in plant production. 1. Inoculum production. Z. Pflanzenkr. Pflanzenschutz. 93: 415-424.

10) Hall, I.F. and A. Kelson. 1981. An Improved technique for the production of endomycorrhizal infested soil pellets. N.Z.J. Agric. Res. 24.

11) Burton, J.C. 1980. New developments in inoculating legumes. pp. 380-405. In N.S. Subba Rao (ed.). Recent advances in Biological Nitrogen Fixation. Edward Arnold Ltd., London.

12) Warner, A. 1983. Mycorrhizal Seed Pellets. U.S. Patent No. 4,551,165.

13) Dommergues, Y.R., H.G. Diem, and C. Divies. 1979. Polyacrylamide-entrapped Rhizobium as an inoculant for legumes. Appl. Environ. Microbiol. 37: 779-781.

14) Bhattacharyya, S.K. and M.K. Basu. 1982. Kaolin powder as a fungal carrier. Appl. Environ. Microbiol. 44: 751-753 15)

15) Kremer, R.J. and H.L. Peterson. 1982. Effect of inoculant carrier on survival of rhizobium on inoculated seed. Soil Sci. 134: 117-125

16) Jung, G., J. Mugnier, H.G. Diem, and Y.R. Dommergues. Polymer-entrapped rhizobium as an inoculant for legumes. Plant Soil 65: 219-231.

17) Gaunt, J.R. 1978. Inoculant of vesicular-arbuscular mycorrhizal fungi on onion and tomato seeds. N.Z.J. Bot. 16: 69-71.

18) Huels, A.G. 1985. Manufacturing and use of adsorbants to inoculate plants with vesicular-arbuscular mycorrhizal fungi. EPO Patent Application No. 0163840.

19) Crawford, S.L. and D.L. Berryhill. 1983. Survival of Rhizobium phaseoli in coal-based legume inoculants applied to seeds. Appl. Environ. Microbiol. 45: 703-705.

20) Hayman, D.S., E.J. Morris, and R.J. Page. 1981. Methods for inoculating field crops with mycorrhizal fungi. Ann. Appl. Biol. 99: 247-253.

21) Johnson, C.R. and R.L. Hummel. 1985. Hydrophylic polymers as a carrier for VA mycorrhizal inoculum. J. Environ. Hort. 3: 166-168.

22) Menge, J.A. 1984. Inoculum production. pp. 187-203. In C.L. Powell and D.J. Bagyaraj (eds.). VA Mycorrhiza. CRC Press, Boca Raton.

23) Mugnier, J., G. Jung, and J. Prioul. 1986. Method for producing endomycorrhizian fungi with arbuscules and vesicles in vitro. U.S. Patent No. 4,599,312.

24) Wood, T.E., H.C. Granger, and B. Biermann. 1985. Method for producing axenic vesicular-arbuscular mycorrhizal fungi in association with root organ cultures. U.S. Patent Application.

25) Tommerup, I.C. and D.K. Kidby. 1980. Production of asceptic spores of vesicular-arbuscular endophytes and their viability after chemical and physical stress. Appl. Environ. Microbiol. 39: 1111-1119.

26) Fawcett, R.G. and N. Collis-George. 1967. A filter-paper method for determining the moisture characteristics of soil. Aust. J. Expt. Agric. Anim. Husb. 7: 162-167.

27) Hamblin, A.P. 1981. Filter-paper method for routine measurement of field water potential. J. Hydrol. 53: 355-360.

28) Tommerup, I.C. and D.K. Kidby. 1979. Preservation of spores of vesicular-arbuscular endophytes by L-drying. Appl. Environ. Microbiol. 37: 831-835.

29) Tommerup, I.C. 1983. Spore dormancy in vesicular-arbuscular mycorrhizal fungi. Trans. Brit. Mycol. Soc. 81: 37-45.

## Claims

1. A method for producing an inoculant comprising viable microorganisms having a degree of dryness selected to prevent undue contamination of the inoculant and premature germination of the microorganisms which includes combining the microorganisms and a water absorbent material at a rate sufficient to attain the selected degree of dryness while maintaining viability of the microorganisms.

2. A method according to claim 1 further comprising the step of separating the viable microorganisms from the water absorbent material.

3. A method according to claim 2 further comprising the step of contacting the viable microorganisms separated from the water absorbent material with a carrier material.

4. A method according to claim 1 wherein the water absorbent material is suitable for use as a carrier for the microorganisms.

5. An inoculant composition comprising a carrier and viable microorganisms dried at a selected rate to a selected degree of dryness.

6. A method according to any one of claims 1 to 4 or a composition according to claim 5 wherein the microorganisms are fungi.

7. A method or composition according to claim 6 wherein the fungi are vesicular-arbuscular mycorrhizal fungi.

8. A method or composition according to claim 7 wherein the vesicular-arbuscular mycorrhizal fungi are selected from members of the genuses Glomus or Gigaspora.

9. A method or composition according to claim 8 wherein the fungi of the genus Glomus are selected from G. intraradices, G. deserticola, G. etunicatum and G. mosseae or the fungi of the genus Gigaspora are G. margarita.

10. A method according to any one of claims 1 to 9 or a composition according to any one of claims 5 to 9 wherein the microorganisms are disinfested.

11. A method according to any one of claims 1 to 10 or a composition according to any one of claims 5 to 10 wherein the water absorbent material or carrier is granular or powdered.

12. A method or composition according to claim 11 wherein the water absorbent material or carrier is clay.

13. A method or composition according to claim 12 wherein the clay is granular and is selected from attapulgite, vermiculite and calcined montmorillinite.

14. A method or composition according to claim 11 wherein the water absorbent material or carrier is powdered and selected from talc, clay, and diatomaceous earth.

15. A method according to any one of claims 1 to 14 or a composition according to any one of claims 5 to 14 wherein the water absorbent material or carrier is treated with a fertilizer material.

16. A method according to any one of claims 1 to 15 or a composition according to any one of claims 5 to 15 wherein the viable micororganisms are bound, by use of an adhesive, to the water absorbent material or carrier.

17. A method or composition according to claim 16 wherein the adhesive is carboxymethyl cellulose.

18. A method according to any one of claims 1 to 17 or a composition according to any one of claims 5 to 17 wherein the degree of dryness is at least -3 MPa.

19. A method or composition according to claim 18 wherein the selected degree of dryness is from -50 to -100 MPa.

20. A method according to any one of claims 1 to 19 wherein the combination rate is selected so as to maximize the viability of the microorganisms.

21. A method according to any one of claims 1 to 20 wherein the microorganisms and water absorbent material are combined at a rate sufficient to maintain the viability of the microorganisms such that they may colonize a plant and form a beneficial association with that plant.

22. A method according to any one of claims 1 to 21 wherein the viability is from 50% to 100%.

23. A method according to any one of claims 1 to 22 or a composition according to any one of claims 5 to 19 wherein the microorganism is Glomus intraradices, and (i) the water absorbent material is a sand-loam substrate, and the rate of combining is sufficient to dry the G. intraradices to a matric potential of -100 MPa in 3 to 16 days, or (ii) water absorbent material is attapulgite, and the rate of combining is sufficient to dry the G. intraradices to a matric potential of -50 MPa in 3 to 7 days, or (iii) water absorbent material is calcined montmorrillinite and the rate of combining is sufficient to dry the G. intraradices to a matric potential of -100 MPa in 8 to 16 days, or (iv) the carrier is sand-loam, and the selected rate of drying is sufficient to obtain a matric potential of from -50 MPa to -100 MPa in 6 to 16 days, or (v) the carrier is attapulgite, and the selected rate of drying is sufficient to obtain a matric potential of from -50 MPa to -100 MPa in 3 to 16 days or (vi) the carrier is calcined montmorrillinite and selected rate of drying is sufficient to obtain a matric potential of from -50 MPa to -100 MPa in 3 to 16 days, or (vii) the carrier is attapulgite, and the selected rate of drying is sufficient to obtain a matric potential of from -50 MPa to -100 MPa in from 1 hour to 7 days.

24. A method according to any one of claims 1 to 22 or a composition according to any one of claims 5 to 19 wherein the microorganism is Glomus deserticola, and (i) the water absorbent material is a sand-loam substrate, and the rate of combining is sufficient to dry the G. deserticola to a matric potential of -100 MPa in 14 to 16 days, or (ii) the water absorbent material is attapulgite, and the rate of combining is sufficient to dry the G. deserticola to a matric potential of -50 MPa in 14 days or (iii) the carrier is attapulgite, and the selected rate of drying is sufficient to obtain a matric potential of from -50 MPa to -100 MPa in 14 days.

25. A method according to any one of claims 1 to 22 or a composition according to any one of claims 5 to 19 wherein the microorganism is Glomus etunicatum, and (i) the water absorbent material is a sand-loam substrate, and the rate of combining is sufficient to dry the G. etunicatum to a matric potential of -100 MPa in from 1 to 3 to 14 to 16 days, or (ii) the water absorbent material is attapulgite, and the rate of combining is sufficient to dry the G. etunicatum to a matric potential of -50 MPa in from 1 hour to 3 days, or (iii) the carrier is attapulgite, and the selected rate of drying is sufficient to obtain a matric potential of from -50 MPa to -100 MPa in from 1 hour to 3 days.

26. A method according to any one of claims 1 to 22 or a composition according to any one of claims 5 to 19 wherein (i) the microorganism is Glomus mosseae, the water absorbent material is a sand-loam substrate, and the rate of combining is sufficient to dry the G. mosseae to a matric potential of -100 MPa in 6 to 16 days, or (ii) the microorganism is Gigaspora margarita, the water absorbent material is a sand-loam matrix, and the rate of combining is sufficient to dry the G. margarita to a matric potential of -100 MPa in 23 days.

27. An inoculant composition comprising a carrier and viable microorganisms dried at a selected rate to a selected degree of dryness.

28. An inoculant according to claim 27 wherein the micororganisms have a selected degree of viability.

29. An inoculant according to claim 27 or 28 comprising a composition according to any one of claims 5 to 19 and 23 to 26 or produced according to any one of claims 1 to 26.

30. Use of an inoculant according to any one of claims 27 to 29 wherein the inoculant is applied to soil, a plant, or a seed in an agriculturally effective amount.

31. Use of an inoculant according to claim 30 wherein the inoculant is applied as a soil amendment, as a bare root treatment, as a seed coating or as a root dip.

MATRIC POTENTIAL VERSUS MOISTURE CONTENT CURVES FOR TURFACE™ & ATTAPUGATE

ATTAPULGATE

TURFACE

MATRIC POTENTIAL (~MPa)

MOISTURE CONTENT (%ODW)

FIG. 1

EP 0 314 439 A2

SEMI-LOG INOCULUM DRYING PLOTS FROM -0.26 MPa TO -50.00 MPa EXTENDING OVER PERIODS OF 3,7 & 14 DAYS

-50 MPa

MATRIC POTENTIAL (-MPa)

TIME (DAYS)

Fig_2_

EP 0 314 439 A2